# EUROPEAN PATENT SPECIFICATION

(11) **EP 2 538 803 B1**
(45) Date of publication and mention of the grant of the patent: **29.03.2017**
(21) Application number: 11706451.9
(22) Date of filing: 24.02.2011
(51) Int. Cl.: A61P 1/00, A61K 31/195, A61K 31/202, A23L 29/10, A23L 33/12, A23L 33/15, A23L 33/16, A23L 33/175, A23L 33/18, A23L 33/19, A23L 33/21

(54) **FORMULATIONS AND METHODS FOR NUTRIENT DELIVERY**
FORMULIERUNGEN UND VEFAHREN ZUR NÄHRSTOFFVERABREICHUNG
FORMULATIONS ET PROCÉDÉS POUR L'ADMINISTRATION DE NUTRIMENTS

(30) Priority: 24.02.2010 US 711376
(43) Date of publication of application: 02.01.2013
(73) Proprietor: MJN U.S. Holdings LLC, Glenview, IL 60026 (US)
(72) Inventor: ALVEY, John, Evansville Indiana 47711 (US); GONZALEZ, Juan M., Newburgh Indiana 47630 (US); TAYLOR, Bradley J., C.P. 11000 Mexico D.F. (MX); MORRIS, Kristin L., Evansville Indiana 47720 (US); ANTHONY, Joshua C., Evansville Indiana 47711 (US); TUCKER, Hugh N., Brevard North Carolina 28712 (US); POELS, Eduard K., Evansville Indiana 47711 (US)
(74) Representative: V.O.
(86) International application number: PCT/US2011/026000
(87) International publication number: WO 2011/106482

(56) References cited:
- WO-A1-2008/056983
- US-A- 4 920 098
- US-A- 5 053 387
- US-A- 5 576 351
- US-A1- 2004 091 598
- US-A1- 2006 083 824
- US-A1- 2009 192 226
- TONY E HAYNES ET AL: "l-Glutamine or l-alanyl-l-glutamine prevents oxidant or endotoxin-induced death of neonatal enterocytes", AMINO ACIDS ; THE FORUM FOR AMINO ACID AND PROTEIN RESEARCH, SPRINGER-VERLAG, VI, vol. 37, no. 1, 3 February 2009 (2009-02-03), pages 131-142, XP019723399, ISSN: 1438-2199

## Description

### TECHNICAL FIELD

The present disclosure relates to improved enteral nutritional formulations comprising one or more of arginine, glutamine, docosahexaenoic acid (DHA) and arachidonic acid (ARA) and also to methods for providing small-volume nutritional support in the form of water soluble and lipid soluble nutrients to a population of subjects suffering from nutritional deficiencies, such as preterm infants, as specified in the appended claims. The nutritional formulation may be suitable for delivery via nasogastric tube, intragastric feeding, transpyloric administration and/or any other means of administration that result in the introduction of the nutritional formulation into the digestive tract of a subject.

### BACKGROUND ART

The present disclosure relates to an improved enteral nutritional formulation that addresses nutritional deficiencies in critically ill populations as well as physiological and other consequences often arising from those deficiencies. In particular, the disclosure addresses nutritional deficiencies that may arise in subjects receiving partial or total parenteral nutrition.

Nutritional support of the premature infant is of great importance since short-term survival and long-term growth and development are at stake. Important goals when providing nutritional support to preterm infants include promoting growth rates and nutrient accretion that are equivalent to those achieved during fetal development, optimizing neurodevelopmental outcomes and laying strong foundations for long-term health. However, these goals are not easily attained, as the critically ill, low birth weight premature infant often cannot tolerate traditional enteral feeding due to concomitant pathologies or immaturity of the intestinal tract and other organ systems. Thus, total parenteral nutrition (TPN) is indicated as either the only or the preferred method of providing nutrition support. And although TPN can be life saving, it is not a perfect means of nutritional support. TPN lacks many critical nutrients, and its limitations may have long-lasting physiological and developmental consequences for infants.

Indeed, TPN fails to provide valuable nutrients, such as glutamine. Glutamine has been traditionally classified as a "non-essential" amino acid because it can be endogenously synthesized in nearly all tissues. Glutamine is important in development because it is the primary fuel for rapidly dividing cells, such as intestinal enterocytes and lymphocytes.

In healthy subjects consuming a normal diet there is no need for glutamine supplementation. However, in critically ill patients experiencing severe catabolism, such as very ill and/or premature infants, intracellular glutamine stores may become depleted, and biosynthetic pathways frequently cannot meet the increased demands of glutamine-metabolizing tissues; glutamine thus becomes a "conditionally essential" amino acid.

In preterm birth, a premature infant faces the sudden loss of the placental glutamine supply. In addition, such infants are often critically ill and face numerous physiological stresses that may rapidly exhaust their available glutamine stores. This problem is exacerbated by the absence of glutamine in parenteral nutrition and TPN amino acid solutions. Parenteral nutritional sources often lack glutamine due to its instability in solution.

Likewise, there are several factors placing premature infants receiving TPN support at risk for arginine deficiency. Arginine is an essential amino acid for the maximal growth of young mammals. L-arginine is synthesized from glutamine, glutamate and proline via the intestinal-renal axis in humans. More specifically, citrulline is synthesized from glutamine, glutamate and proline in the mitochondria of intestinal enterocytes, released from the small intestine, and taken up primarily by the kidneys for arginine production. In human infants, most of the citrulline synthesized in enterocytes is converted locally into arginine. L-arginine is the substrate for the synthesis of nitric oxide (NO), a potent vasodilator in the systemic, gastrointestinal and pulmonary circulation. Endothelial nitric oxide (NO) is an important regulator of vascular perfusion, and NO exerts anti-inflammatory and vasodilatory actions which are involved in the maintenance of mucosal integrity, intestinal barrier function, regulation of intestinal mucosal blood flow in the face of inflammation or injury and the normal transition from fetal to neonatal circulation.

Arginine is also required for the detoxification of ammonia. Consequently, life-threatening hyperammonemia can occur in premature infants as a result of arginine deficiency. Premature infants have underdeveloped arginine-synthetic pathways and reduced intestinal mass for citrulline, consequently having reduced capacity for endogenous arginine production and requiring arginine supplementation to their diets. Importantly, then, supplemental arginine may prevent hyperammonemia in premature infants receiving TPN.

Furthermore, stress frequently results in deprivation of both arginine and glutamine in premature infants undergoing intensive care. (Neu J. Glutamine supplements in premature infants: why and how. J Pediatr Gastroenterol Nutr. 2003;37(5):533-535.) Yet, as previously discussed, commercial TPN solutions do not contain glutamine, nor do they include sufficient amounts of arginine.

Nonetheless, evidence suggests that arginine is an essential amino acid for premature infants receiving TPN. For example, premature infants with necrotizing entercolitis and persistent pulmonary hypertension have decreased plasma arginine concentrations, and arginine availability may be an important factor in limiting NO formation in this population. Plasma L-arginine levels were shown to be low in premature infants and associated with severity of respiratory distress syndrome. Plasma L-arginine levels were also found to be low in premature infants at the time of diagnosis with necrotizing entercolitis. Accordingly, arginine supplementation in low-birth-weight infants shows a reduction in levels of necrotizing entercolitis possibly due to improvement in blood flow to the microvasculature of the intestine due to increased local nitric oxide production through the L-arginine-nitric oxide synthase pathway. (Amin HJ, Zamora SA, McMillan DD, Fick GH, Butzner JD, Parsons HG, Scott RB. Arginine supplementation prevents necrotizing entercolitis in the premature infant. J Pediatr. 2002;140(4):425-431.) And glutamine and arginine supplementation have both been shown to be safe in low-birth-weight infants in multicenter trials of intravenous and enteral glutamine supplementations. (Poindexter BB, Ehrenkranz RA, Stoll BJ, Wright LL, Poole WK, Oh W, Bauer CR, Papile LA, Tyson JE, Carlo WA, Laptook AR, Narendran V, Stevenson DK, Fanaroff AA, Korones SB, Shankaran S, Finer NN, Lemons JA. Parenteral glutamine supplementation does not reduce the risk of mortality or late-onset sepsis in extremely low birth weight infants. Pediatrics. 2004;113(5):1209-1215 and Vaughn P, Thomas P, Clark R, Neu J. Enteral glutamine supplementation and morbidity in low birth weight infants. J Pediatr. 2003;142(6):662-668.)

It is well known that proteins are converted to amino acids in the digestive system and that the resulting amino acids are used by the body for growth and development. Proteins and peptides administered for therapeutic or preventative measures are also well-known. Oligopeptides are better absorbed in the intestines than individual amino acids. Accordingly, arginyl-glutamine or alanyl-glutamine may be utilized as a dipeptide source of arginine and glutamine, rather than the individual amino acids arginine and glutamine, in order to improve stability of a formula or due to the increased solubility and absorption capacity of the dimers over the monomers.

TPN and other parenteral nutritional supplements also provide negligible amounts of preformed DHA and ARA. DHA is an omega-3-fatty acid and is the most abundant long chain polyunsaturated fatty acid (LCPUFA) in the brain and retina and is thought to be essential for proper brain and vision development of infants. Although a metabolic pathway exists for biosynthesis from dietary linolenic acid, the pathway is bioenergetically unfavorable, and mammals obtain most of their DHA from preformed DHA provided via dietary sources. For infants, then, the source of DHA is typically human milk; however, DHA is typically absent from parenteral formulas provided to preterm infants.

Parenteral formulas generally fail to provide sufficient amounts of arachidonic acid (ARA) as well. ARA is an omega-6 LCPUFA that serves a major role as a structural lipid associated with phospholipids in the blood, liver, muscle and other major organ systems. ARA is synthesized by the elongation and desaturation of linoleic acid. Yet, most ARA must be provided in the diet. ARA is especially important during periods of rapid body growth, and is, therefore, an important component of infant nutrition.

Numerous studies have indicated that unsupplemented preterm milk provided to infants provides inadequate quantities of several nutrients to meet the needs of preterm infants (Davis, D.P., "Adequacy of expressed breast milk for early growth of preterm infants", Archives of Disease in Childhood, 52, p. 296-301, 1997). While exact needs vary among infants due to differences in activity, energy expenditure, efficiency of nutrient absorption, illness and the ability to utilize energy for tissue synthesis, presently available parenteral nutritional sources are inadequate.

Moreover, feeding volume is often not well tolerated in preterm infants, and nutrients must be provided in an acceptable volume, often via enteral administration. An appropriate method of enteral feeding for a preterm infant is based on gestational age, birth weight, clinical condition and on the opinion of presiding medical personnel. Specific feeding decisions are made based on an infant's ability to coordinate sucking, swallowing and breathing. Frequently, preterm infants or infants who are less mature, weak or critically ill require feeding by tube to avoid risks of aspiration and to conserve energy.

Nasogastric feedings are commonly used in neonatal intensive care units and may be accomplished with bolus or continuous infusions of fortified human milk or other nutritional supplements. Continuous feedings may be better tolerated by very low birth weight infants and infants who have not previously tolerated bolus feedings; however, as previously discussed, reduced or deficient nutrient delivery is a problem associated with continuous feeding methods known in the art.

Therefore, there is a need for stable nutritional formulations and methods that are well tolerated by preterm infants and that can be easily administered to subjects suffering from nutritional deficiencies in forms and manners that are readily accepted by the subject and the caregiver.

Populations, such as preterm infants, often suffer nutritional deficiencies because they are provided with diets lacking critical nutrients as described above. Thus, a need exists in the art to provide a nutritional formulation comprising valuable nutrients that support infant development, such as DHA, ARA, arginine and glutamine. Therefore, the nutritional formulations and methods of the present disclosure provide enteral nutritional support to subjects suffering from nutritional deficiencies in order to promote optimum health and development by delivering important nutrients that are either absent from or provided in inadequate amounts in known parenteral nutrition formulas.

### DISCLOSURE OF THE INVENTION

Briefly, therefore, the present disclosure is directed to a stable nutritional formulation for addressing nutritional deficiencies in subjects, such as preterm infants, requiring small-volume nutritional support as specified in claim 1, and to methods for providing nutritional support as specified in claims 5 and 9.

In one embodiment, the present disclosure comprises a nutritional formulation, comprising an emulsion of docosahexaenoic acid (DHA) dispersed in an aqueous component comprising at least one of an amino acid component selected from the group consisting of arginine, arginyl-glutamine, and alanyl-glutamine and a surfactant comprising highly-purified α-lactalbumin.

In another embodiment, the present disclosure comprises a method for providing nutritional support to a subject, the method comprising administering to the subject a nutritional formulation comprising an emulsion of docosahexaenoic acid dispersed in an aqueous component comprising at least one of an amino acid component selected from the group consisting of arginine, arginyl-glutamine, and alanyl-glutamine, and a surfactant comprising highly-purified α-lactalbumin.

Yet another embodiment comprises a method for providing nutritional support to preterm infants, wherein the method comprises enterally administering to a preterm infant a nutritional formulation comprising an emulsion of docosahexaenoic acid dispersed in an aqueous component comprising at least one of an amino acid component selected from the group consisting of: arginine, arginyl-glutamine, and alanyl-glutamine, along with an emulsifier comprising at least about 95% w/w α-lactalbumin.

It is to be understood that both the foregoing general description and the following detailed description present embodiments of the disclosure and are intended to provide an overview or framework for understanding the nature and character of the disclosure as it is claimed. The description serves to explain the principles and operations of the claimed subject matter. Other and further features and advantages of the present disclosure will be readily apparent to those skilled in the art upon a reading of the following disclosure.

### BRIEF DESCRIPTION OF THE DRAWINGS

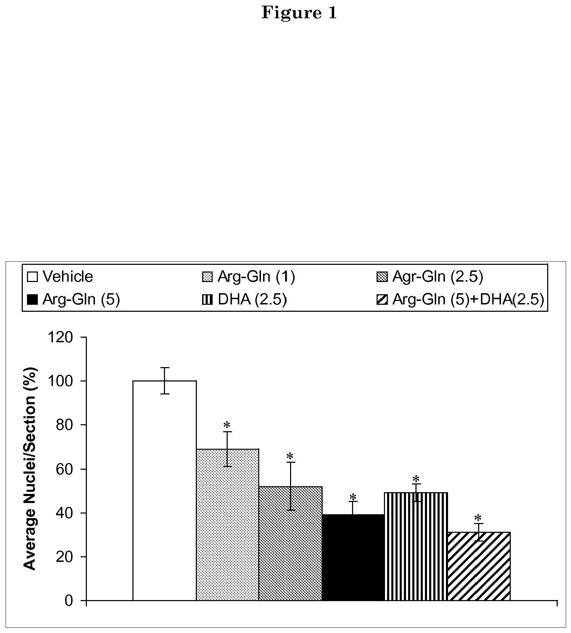

Fig. 1 is a bar chart showing the synergistic effect of administration of the Arg-Gln dipeptide and DHA to promote healthy eye development by reducing pre-retinal neovascularization in a mouse model. Fig. 1 provides a graphical summary of the analysis of pre-retinal neovascularization levels in the mouse model following gavage treatments. The numbers in brackets in the legend refer to gavage doses in g/kg bodyweight/day. An * indicates P-values < 0.05.

### BEST MODE FOR CARRYING OUT THE INVENTION

The present disclosure provides nutritional formulations and methods for providing nutritional support in the form of water soluble and lipid soluble nutrients to any population of subjects suffering from nutritional deficiencies, such as preterm infants. A full and enabling disclosure of the present invention(s), including the best mode thereof directed to one of ordinary skill in the art, is set forth in the specification below.

The present disclosure further provides an improved enteral nutritional formulation for correcting nutritional deficiencies and a method for providing enteral nutritional support to a subject in the form of water soluble and lipid soluble nutrients. The present disclosure provides formulations for administering critical nutrients, such as DHA and ARA, amino acids, such as arginine and glutamine, and other nutrients in order to prevent development of nutritional deficiencies, to correct existing nutritional deficiencies, and/or to promote healthy development of a subject.

Hereinafter, "emulsion" means a mixture of two or more immiscible liquids comprising a dispersed phase and a continuous phase. In an emulsion, one liquid, called the dispersed phase, is dispersed in the other liquid, called the continuous phase, bulk phase, or aqueous component.

"Surfactant" or "emulsifier" means surface active substance that can increase the stability of an emulsion. The surfactant is positioned on the interface between the dispersed phase and the aqueous phase of an emulsion. Surfactants may increase the stability of an emulsion so that, once formed, the emulsion does not separate over years of storage.

"Preterm infant" means a subject born before 37 weeks gestational age. The phrase "preterm infant" is used interchangeably with the phrase "premature infant."

"Low birth weight infant" means an infant born weighing less than 2500 grams (approximately 5 lbs, 8 ounces).

"Very low birth weight infant" means an infant born weighing less than 1500 grams (approximately 3lbs, 4 ounces).

"Infant" means a subject ranging in age from birth to not more than about one year and includes infants from 0 to about 12 months corrected age. The term infant includes low birth weight infants, very low birth weight infants, and preterm infants.

All percentages, parts and ratios as used herein are by weight of the total formulation, unless otherwise specified.

The nutritional formulation of the present disclosure may also be substantially free of any optional or selected ingredients described herein, provided that the remaining nutritional formulation still contains all of the required ingredients or features described herein. In this context, and unless otherwise specified, the term "substantially free" means that the selected formulation contains less than a functional amount of the optional ingredient, typically less than 0.1% by weight, and also, including zero percent by weight of such optional or selected ingredient.

All references to singular characteristics or limitations of the present invention shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The methods and formulations of the present invention, including components thereof, can comprise, consist of, or consist essentially of the essential elements and limitations of the invention described herein, as well as any additional or optional ingredients, components or limitations described herein or otherwise useful in nutritional formulations.

As used herein, the term "about" should be construed to refer to both of the numbers specified in any range. Any reference to a range should be considered as providing support for any subset within that range.

The nutritional formulation of the present disclosure may provide nutritional support and personalized nutrition to preterm infants, to infants or to any other patient with unmet nutritional needs. Thus, in some embodiments, the nutritional formulation is designed to meet specific nutritional needs of individual subjects, such as infants or preterm infants, in stable, unit-dose liquid formulations standardized to a caloric content and/or as a concentrate to meet a subject's particular nutritional needs.

Furthermore, the present disclosure provides a nutritional formulation to combat nutritional inadequacy in the provision of nutrients to those relying on parenteral or total parenteral nutrition, thereby promoting healthy development of a subject. Indeed, the nutritional formulation provides nutrients, such as DHA, that will promote, for example, visual and neural development in an infant.

The present disclosure also provides a method for enterally delivering nutrients to a subject who requires that the nutrients be administered in very small volumes. As used herein, enteral administration includes, feeding via nasogastric tube, intragastric feeding, transpyloric administration or any other method known in the art for introducing a nutritional formulation directly into the digestive tract.

Thus, the present disclosure addresses the needs of any population that may require small volume enteral nutrition support, including but not limited to perisurgical subjects, subject with short-gut syndrome, pediatric intensive care subjects, and/or any population of any age that is unable to fully orally feed or that is receiving minimal enteral nutrition support or TPN. Additionally, in some embodiments, the present invention may provide nutritional support to companion animals or to non-human primates.

Specifically, the nutritional formulation of the present disclosure may provide infants with beneficial nutrients which are otherwise absent due to a variety of conditions, such as prematurity or trauma.

The present disclosure is directed to a nutritional formulation that delivers, in a small volume, a set of specific nutrients to a subject. The resulting nutritional formulation may be commercially viable and is practical for use in critical care settings including, but not limited to, the neonatal intensive care unit (NICU). In some embodiments, the nutritional formulation of the present disclosure comprises an enteral nutrient delivery system whereby small but precise volumes of nutritional formulations are introduced into the digestive tract of a subject. In some embodiments, the nutritional formulation is delivered in a volume dose of about 1 mL. In another embodiment, the nutritional formulation may be delivered in volumes up to about 1.5 mL or about 2 mL.

In some embodiments, the nutritional formulation delivers valuable nutrients to a preterm infant or infant in a small-volume liquid dose of about 1 mL. While preterm infants are often too ill to tolerate full enteral feeds, the nutritional formulations of the present disclosure are designed to be administered as a small-volume nutritional supplement that may be directly administered to an infant through, for example, a nasogastric tube that is placed in all infants in the NICU. Thus, administration of the presently disclosed nutritional formulation may begin on the first day postpartum.

Moreover, the nutritional formulation may be administered one to two times daily or more frequently as directed by a medical professional. Administration may begin immediately after birth and may continue as long as a subject is in nutritional need.

DHA and ARA are incorporated into the nutritional formulation.

In some embodiments the nutritional formulation may comprise between about 5 and about 20% w/w lipids. Further, in one embodiment, the nutritional formulation comprises a source of DHA comprising DHASCO® and/or a fungal oil blend. The nutritional formulation may comprise between about 1 and about 5% w/w DHA in some embodiments. In some embodiments, the DHASCO® comprises about 40% DHA, and the fungal oil blend comprises about 15% DHA. The source of DHA may be any source known in the art.

In an embodiment, the nutritional formulation comprises a source of ARA comprising ARASCO® and/or a fungal oil blend. In some embodiments, the ARA component of the nutritional supplement comprises about 30% of a fungal oil blend. The nutritional formulation may comprise between about 1% and about 5% w/w ARA in some embodiments. The ARA source may be any source of ARA known in the art.

Formulas known in the art are prone to physical instability due to syneresis and to the formation of non-dispersible sediments. Instability is caused by the high levels of protein, fats and minerals that known nutritional formulas must contain in order to provide adequate nutrition in a reasonable volume. Notably, acidification of traditional enteral formulas may also lead to protein precipitation and phase separation. Precipitated nutrients cannot generally be shaken back into solution, and they do not provide the nutritional benefits required to promote the health of a subject.

However, the nutritional formulation of the present disclosure provides an improved enteral formula that provides an acceptable small-volume unit-dose for preterm infants, contains valuable proteins, amino acids and fatty acids, and has a shelf life of at least about one year due to excellent physical stability. Further, the present disclosure provides a novel stabilizing system that generally prevents nutrients from precipitating or separating from the enteral formula and a method for preparing a nutritional formulation containing the same.

The nutritional formulation of the present disclosure may comprise a stable emulsion further comprising a stabilization agent, also referred to as an emulsifier. The emulsifier may comprise microencapsulants, surfactants, emulsion stabilizers or a combination thereof. In some embodiments, the lipids in the nutritional formulation are in the form of a stable emulsion. Emulsions may be stabilized by several mechanisms that may affect viscosity, density, particle size and surface tension.

In some embodiments, the step of emulsifying may be by mechanical agitation, ultrasonic vibration, heating, or a combination thereof. Emulsification may be accomplished using any method for emulsification known in the art. In one embodiment, emulsification may comprise homogenization. In some embodiments, multiple homogenization steps may be applied.

In some embodiments of the stable emulsion, proteins may act as surfactants. Protein surfactants have the capability of spreading at a lipid-water interface in order to reduce droplet coalescence. Indeed, a protein surfactant may lower the interfacial tension between two liquids resulting in the miscibility of the two liquids. The nutritional formulation may comprise any emulsifier that is water soluble.

The α-lactalbumin acts as a stabilizing agent, specifically as a surfactant. Additional surfactants, emulsion stabilizers and microencapsulants may be used but are not required in order to produce the stable, emulsified nutritional formulation of the present disclosure.

The structure of a-lactalbumin confers the ability to migrate and unfold at a water-lipid interface, thereby creating a thermodynamically stable emulsion. The structure also confers the ability for cooperative binding with itself at the lipid-water interface, thus creating a synergistic surface adsorption effect allowing for formation of strong, stable emulsions.

Some embodiments of the nutritional formulation, such as those that are optimized for preterm infants or critically ill infants, may mimic certain characteristics of human breast milk. Indeed, the nutritional formula may comprise α-lactalbumin, which is the dominant whey protein in human breast milk. Adding α-lactalbumin to a formulation for preterm infants may provide several physiological and nutritional benefits. Likewise, the addition of DHA, ARA, and arginine and glutamine will provide physiological benefits to an infant.

The present disclosure provides a method for utilizing α-lactalbumin as a surfactant to stabilize an emulsion thereby reducing droplet coalescence and consequential separation of the emulsion of the nutritional formulation. In some embodiments, the nutritional formulation comprises between about 0.1% and about 1.0% w/w α-lactalbumin.

The α-lactalbumin of the nutritional formulation may be highly-purified α-lactalbumin. Highly-purified α-lactalbumin contains at least about 90% w/w α-lactalbumin, preferably containing at least about 95% w/w α-lactalbumin and even more preferably containing at least about 98% w/w α-lactalbumin. Use of highly-purified α-lactalbumin for creating a stabilized emulsion system is unique and advantageous, particularly as it applies to preterm and critically ill infants.

Incorporation of α-lactalbumin in the present nutritional formulation may involve creating an aqueous dispersion of α-lactalbumin at a concentration in the range of about 5 to about 30 mg/L in the bulk phase, that is, in an aqueous component such as water. Moreover, the sourced protein may have a concentration greater than about 95% w/w and an α-lactalbumin concentration greater than about 90% w/w. In some embodiments, the nutritional formulation comprises between about 0.1% and about 1.0% w/w α-lactalbumin.

The purity of the α-lactalbumin is critical, as one of the objectives of the invention is to provide a small-volume nutrient delivery formulation. Thus, it is imperative that ingredient selection for the nutritional formulation be targeted to those nutrients that are most physiologically relevant. The most highly-purified fraction of α-lactalbumin available for the purposes of creating the emulsion should be used in order to minimize the likelihood that increasing the amount of α-lactalbumin required to stabilize the emulsion might ultimately adversely impact the efficacious dose of physiologically relevant nutrients that can be included in a small volume portion or dose of the nutritional formulation.

Furthermore, selection of highly-purified α-lactalbumin enhances hypoallergenicity of the nutritional formulation. As the purity of a protein substrate declines, the likelihood of inclusion of allergens, such as β-lactoglobulin, increases. A subject ingesting the presently-described nutritional formulation may be a preterm infant or a critically ill patient having very immature gut. Therefore, exposure to any allergens or impurities may stress the subject's immune response, and any such reaction is undesirable. So the purest protein available should be used in order to avoid an undesirable immune response in a subject. The protein substrate utilized in the nutritional formulation may comprise pretreated or hydrolyzed protein. The protein substrate may have been hydrolyzed by a method of enzymatic hydrolysis, chemical disruption, physical-mechanical disruption, nonmechanical disruption or combinations thereof.

The pH of the aforementioned dispersion may be adjusted to levels near the isoelectric point of about 4.2 to about 4.5 using an acidulant. Food acids, such as citric acid, may be used as acidulants to adjust the pH of the nutritional formulation. Acidulants that may be used in the present nutritional formulation include, but are not limited to, citric acid and phosphoric acid.

The lipid phase of the dispersion, such as DHA, may be added to the dispersion at a concentration in the range of about 75 to about 300 mg per 100 mL in order to deliver a target of about 34 mg of the lipid component per day to a subject. In some embodiments, the nutritional formulation comprises between about 1 and about 5% w/w DHA.

The aqueous element comprising the bulk-phase of the emulsion may be any suitable substance known in the art. In one embodiment, the bulk phase of the emulsion comprises water.

To achieve a stabilized emulsion, the emulsification process may be carried out using a single stage homogenizer with flux rates up to about 250 mL/min and between about 5000 to about 15,000 psi at temperatures ranging from between about 2°C and about 40°C, ± 2°C.

High pressures are applied to the dispersion in order to provide for homogenization. Sonocation may be used to disperse the α-lactalbumin and force it into the interface of the emulsification droplets as they form.

The emulsion obtained according to the process described above contains oil/lipid droplets ranging from about 0.070 µm to about 1µm in diameter.

Other nutrients and ingredients, such as amino acids, vitamins, and minerals may be incorporated into the phase, or aqueous element, of the emulsion. It may be advantageous to add such other ingredients directly by mixing into the emulsion after homogenization. Indeed, the α-lactalbumin stabilized emulsion allows for the incorporation of other nutrients into the aqueous element without desorption, disruption, or coalescence of the lipid droplets.

Moreover, in some embodiments, the nutritional formulation comprising the emulsion, is nutritionally complete, containing suitable types and amounts of lipids, carbohydrates, proteins, vitamins and minerals to be a subject's sole source of nutrition.

The formulation may further comprise essential amino acids, such as arginine and/or glutamine. The amino acids may be provided in any form that can be ingested and absorbed. Accordingly, arginyl-glutamine or alanyl-glutamine may be utilized as a dipeptide source of arginine and glutamine, rather than the individual amino acids arginine and glutamine, in order to improve stability of a formula or due to the increased solubility and absorption capacity of the dimers over the monomers.

In some embodiments, the nutritional formulation comprises glutamine-glutamine dipeptide, glycyl-glutamine dipeptide, N-acetyl-glutamine or other aqueous stable glutamine analogues.

Arginine is an essential amino acid for infants. Decreased plasma levels of arginine may reflect respiratory distress syndrome or may be associated with necrotizing entercolitis; however, the symptoms of necrotizing entercolitis may be alleviated by arginine supplementation. As such, the nutritional formulation according to the present disclosure may comprise up to about 250 mg/mL arginine. In some embodiments, the formulation comprises less than about 225 mg/mL arginine, and in other embodiments, the formulation comprises less than about 216 mg/mL arginine.

Moreover, in some embodiments, the nutritional formulation may comprise between about 15 and about 20% w/w of arginine. In one embodiment, the nutritional formulation provides 500mg/kg/day arginine to a subject.

Likewise, glutamine has important effects on immune function and is particularly beneficial to intestinal cells. Moreover, low birth weight infants receiving glutamine may have reduced need for mechanical ventilation. Thus, the nutritional formulation may comprise an aqueous-stable form of glutamine, which may be delivered in the form of an alanyl-glutamine dipeptide or an arginyl-glutamine dipeptide. Alanyl-glutamine dipeptide may be preferred over arginyl-glutamine dipeptide due to its commercial availability and relatively efficient synthesis process.

In one embodiment, it is useful to provide arginine and glutamine in the form of arginyl-glutamine dipeptide, which has excellent water solubility and bioavailability to humans and animals. In one form, the arginyl-glutamine dipeptide has an N-terminal amino acid, which is arginine, and a C-terminal amino acid, which is glutamine. The use of the Arg-Gln as a dipeptide, rather than as individual amino acids, is due to improved stability, increased solubility and increased absorption of the dimer over the monomers.

In one embodiment of the invention, the arginine, glutamine, and/or arginyl-glutamine or alanyl-glutamine dipeptides can be used for preventing the proliferation of abnormal retinal blood vessels in an infant or preterm infant. The phrase "dipeptides" refers to, at least, arginyl-glutamine dipeptide and alanyl-glutamine dipeptide. Thus, in some embodiments, the nutritional formulation supports healthy visual development.

In an embodiment, the combination of arginine and glutamine or the arginyl-glutamine dipeptide(s) is administered to a subject in an amount that is effective to prevent abnormal vascular proliferation. This amount can be from about 0.001 to about 10,000 mg/kg-day (where the units of mg/kg-day refer to the mg of the combination of arginine and glutamine in roughly equimolar amounts or mg of the Arg-Gln dipeptide, per kg of subject body weight per day).

The nutritional formulation may comprise less than about 400 mg/mL arginyl-glutamine, comprising up to 375 mg/mL arginyl-glutamine in some embodiments and up to about 387 mg/mL in other embodiments. In certain embodiments, the nutritional formulation may comprise between about 100 and about 400 mg/mL arginyl-glutamine.

The nutritional formulation may comprise less than about 300 mg/mL alanyl-glutamine, comprising up to about 280 mg/mL alanyl-glutamine in some embodiments and as much as 269 mg/mL in other embodiments.

In some embodiments, the nutritional formulation comprises between about 8 and about 12% w/w alanyl glutamine. In one embodiment, the nutritional formulation provides a subject with 300mg/kg/day alanyl glutamine.

Adding nutrients that are of high nutritional value, such as, for example, arginine and alanyl-glutamine dipeptide, to the nutritional formulation after homogenization is preferred in order to minimize any losses of such valuable nutritional components.

Moreover, in some embodiments, the nutritional formulation contains both DHA and Arg-Gln. Embodiments of the nutritional formulation comprising both DHA and Arg-Gln may promote healthy visual and neural development in an infant.

For example, as shown in Fig. 1, the Arg-Gln dipeptide administered along with DHA may exhibit a synergistic effect that significantly reduces pre-retinal neovascularization in infants. Fig. 1 shows the effects of the compounds as administered in an OIR mouse model, displaying the summary of the results of the analysis of pre-retinal neovascularization levels in the OIR mouse model.

Gavage with the Arg-Gln dipeptide showed a dose-dependent reduction in pre-retinal neovascularization similar to the intraperitoneal injection of the Arg-Gln dipeptide with 5g/kg of body weight/day reducing pre-retinal neovascularization to 39 ± 6% (P < 0.05) relative to vehicle control. Gavage with DHA at 2.5g/kg body weight/day also reduced pre-retinal neovascularization to 49 ± 4% (P < 0.05). Gavage with both the Arg-Gln dipeptide (5 g/kg body weight/day) and DHA (2.5 g/kg body weight/day) showed a synergistic effect with reduction of pre-retinal neovascularization to 31 ± 4% (P < 0.05).

Along with the amino acids or dipeptides, the composition of the invention may contain an additional nitrogen source (i.e. other amino acids and/or protein(s)).

The nutritional formulation of the present disclosure may further comprise flavors, flavor enhancers, sweeteners, pigments, vitamins, minerals, therapeutic ingredients, functional food ingredients, food ingredients, processing ingredients or combinations thereof.

The nutritional formulation may also optionally include any number of proteins, peptides, amino acids, fatty acids, probiotics and/or their metabolic byproducts, prebiotics, carbohydrates and any other nutrient or other compound that may provide many nutritional and physiological benefits to a subject. The carbohydrates utilized in the nutritional formulation may be any digestible carbohydrates, such as dextrose, fructose, sucrose, maltose, maltodextrin, corn syrup solids, or mixtures thereof, depending on usage. Hydrolyzed carbohydrates may be desirable due to their easy digestibility.

In certain embodiments, the nutritional formulation of the present disclosure additionally comprises at least one prebiotic. In this embodiment, any prebiotic known in the art may be added. In a particular embodiment, the prebiotic can be selected from the group consisting of fructo-oligosaccharide, glucooligosaccharide, galacto-oligosaccharide, isomalto-oligosaccharide, xylooligosaccharide and lactulose.

The present disclosure further provides a method for producing a nutritional formulation comprising an emulsion. The method includes emulsifying a lipid component with a protein surfactant to form a stable product as previously described. Accordingly, the present disclosure provides methods to minimize the degradation of nutrients, including LCPUFAs, in a stable formulation such as a nutritional formulation.

The nutritional formulation of the present disclosure may be commercially packaged such that it may interface directly with enteral nutrition apparatuses including, but not limited to, nasogastric tubing, percutaneous endoscopic gastronomy, percutaneous endoscopic jejunostomy, transpyloric tubing and the like. Such a design is convenient to ensure full delivery of the package contents, to minimize the risk of contamination and to increase compliance. Further, in certain embodiments, the nutritional formulation may be packaged in a single dose delivery package of about 1 mL total volume, about 1.5 mL total volume, or about 2 mL total volume.

The nutritional formulation may be expelled directly into a subject's intestinal tract. In some embodiments, the nutritional formulation is expelled directly into the gut. In some embodiments, the formulation may be formulated to be consumed or administered enterally under the supervision of a physician and may be intended for the specific dietary management of a disease or condition for which distinctive nutritional requirements, based on recognized scientific principles, are established by medical evaluation.

The nutritional formulation of the present disclosure is not limited to formulations comprising nutrients specifically listed herein. Any nutrients may be delivered as part of the formulation for the purpose of meeting nutritional needs and/or in order to optimize the nutritional status in a subject.

In some embodiments, the nutritional formulation may be delivered to preterm infants from birth until at least about three months corrected age. In another embodiment, the nutritional formulation may be delivered to a subject as long as is necessary to correct nutritional deficiencies. In yet another embodiment, the nutritional formulation may be delivered to an infant from birth until at least about one year corrected age.

The nutritional formulation of the present disclosure may be standardized to a specific caloric content, it may be provided as a ready-to-use product, or it may be provided in a concentrated form.

In one embodiment, the present disclosure provides a method for preparing a nutritional formulation, comprising the steps of (i) creating an aqueous dispersion of between about 1 to about 30 mg/L of a protein substrate comprising at least 90% w/w α-lactalbumin in the bulk phase, (ii) adjusting the pH of the dispersion to between about 4.2 and about 4.5, (iii) adding between about 75 and about 300 mg/100mL of a lipid component, and (iv) emulsifying the lipid component to create droplets of the lipid component ranging in size from about 0.070 to about 1µm in diameter.

In another embodiment, the present disclosure provides a nutritional supplement for enteral administration comprising an emulsion of a nutritive fatty acid in an aqueous component comprising additional nutrients that is designed to meet the nutritional needs of an infant.

The nutritional formulations and methods of the present disclosure provide significant benefits over the prior art by addressing and correcting nutritional deficiencies in currently available products. Further, the nutritional formulation of the present invention provides valuable nutrients to preterm infants who would not otherwise receive such nutrients when relying on existing sources of TPN.

The following examples are provided to illustrate the inventions of the present disclosure but should not be interpreted as a limitation thereon.

### EXAMPLES

Table 1 provides four example embodiments of the liquid nutritional formulation according to the present disclosure. Concentrations of each ingredient are listed in the Table and have units of g/kg/day. Further, each formulation described in Table 1 has been normalized to be equimolar with arginyl-glutamine. The embodiments of the nutritional formulation described in Table 1 are suitable for administration to animals, such as rodent or piglet models.

**Table 1: Embodiments of the nutritional formulation**

| **Formulation** | **Citric Acid** | **α-lactalbumin** | **DHASCO®** | **Arginyl Glutamine** | **Alanyl Glutamine** | **Arginine** |
|---|---|---|---|---|---|---|
| 1 | 0.03 | 0.01 | 0.25 | 0.00 | 0.72 | 0.58 |
| 2 | 0.00 | 0.01 | 0.25 | 1.00 | 0.00 | 0.00 |
| 3 | 0.04 | 0.01 | 0.25 | 0.00 | 1.80 | 1.44 |
| 4 | 0.00 | 0.01 | 0.25 | 2.50 | 0.00 | 0.00 |

DHASCO® refers to a mixture of oil extracted from the unicellular alga *Crypthecodinium cohnii* and high oleic sunflower oil. The resulting mixed oil contains about 40-45% of product weight as DHA. DHASCO® is commercially available from the Martek Biosciences Corporation.

Table 2 provides another example formulation of the liquid nutritional formulation according to the present disclosure. Table 2 provides an embodiment of the nutritional formulation that is suitable for administration to a human infant. Concentrations of each ingredient are listed in the Table and have units of %w/w.

**Table 2: Embodiment of the nutritional formulation comprising ARA**

| **Citric Acid** | **α-lactalbumin** | **DHASCO®** | **ARASCO®** | **Alanyl Glutamine** | **Arginine** |
|---|---|---|---|---|---|
| 0.67 | 0.7 | 9.3 | 4.6 | 14.6 | 24.3 |

ARASCO® refers to a mixture of an oil extracted from the unicellular fungus *Mortierella alpina* and HOSO and contains 38-33% ARA by weight. ARASCO® contains no detectable levels of eicosapentaenoic acid (EPA) or other LCPUFAs. DHASCO® and ARASCO® are absorbed by healthy infants in the same manner as other dietary triglycerides.

Table 3 provides an embodiment of the nutritional formulation that is optimized for small-volume administration to a human infant. The embodiment of Table 3 may be administered, for example, in a dose of about 1.5 mL, twice daily, to an infant weighing about 1kg who consumes about 100 kcal/day. Concentrations of each ingredient are provided as a range of % w/w.

**Table 3: Embodiment of the nutritional formulation for 1kg human infant**

| **α-lactalbumin** | **DHA** | **ARA** | **Alanyl Glutamine** | **Arginine** |
|---|---|---|---|---|
| 0.28-.70 | 0.8-3.8 | 1.3-1.9 | 12-18 | 20-30 |

More specifically, the nutritional formulation described in Table 3 may comprise about 24.3% arginine, about 14.6% glutamine, about 13.8% of a lipid component, and about 0.69% α-lactalbumin. In another embodiment, the nutritional formulation described in Table 3 may comprise about 0.250g arginine, about 0.150g alanyl glutamine, about 0.0955g DHASCO, about 0.0469g ARASCO, and about 0.007g α-lactalbumin.

In yet another embodiment, the nutritional formulation may be adjusted for delivery to a human infant weighing about 1 kg, wherein the infant receives 2 doses of about 1 mL of the nutritional formulation per day and wherein the infant receives approximately 150 kcal/day. In such an embodiment, the nutritional formulation may comprise about 0.250g arginine, about 0.150g alanyl glutamine, about 0.0955g DHASCO, about 0.0469g ARASCO, and about 0.007g α-lactalbumin.

## Claims

1. A nutritional formulation, comprising:
an emulsion comprising docosahexaenoic acid, wherein the emulsion is dispersed in an aqueous component comprising an amino acid component comprising i) arginine and alanyl-glutamine dipeptide, or ii) arginyl-glutamine dipeptide;
an emulsifier comprising highly-purified α-lactalbumin containing at least 95% w/w of α-lactalbumin, and
arachidonic acid,
wherein the nutritional formulation comprises 0.1 to 1.0 % w/w of the α-lactalbumin and is packaged in a single dose delivery package of up to 2mL.

2. The liquid nutritional formulation of claim 1, wherein the highly-purified α-lactalbumin contains at least 98% w/w of α-lactalbumin.

3. The liquid nutritional formulation of claim 1, further comprising at least one prebiotic.

4. The liquid nutritional formulation of claim 1, further comprising vitamins and minerals.

5. A method for providing nutritional support to a subject, the method comprising administering to the subject a nutritional formulation comprising:
an emulsion comprising docosahexaenoic acid, wherein the emulsion is dispersed in an aqueous component comprising an amino acid component comprising i) arginine and alanyl-glutamine dipeptide, or ii) arginyl-glutamine dipeptide; and
an emulsifier comprising highly-purified α-lactalbumin containing at least 95% w/w of α-lactalbumin, and
arachidonic acid,
wherein the nutritional formulation comprises 0.1 to 1.0 % w/w of the α-lactalbumin, wherein the subject is in need of small volume enteral nutrition support, and wherein the small volume is up to 2 mL per volume dose.

6. The method of claim 5, wherein the emulsifier comprises at least 95% w/w of α-lactalbumin, preferably at least 98% w/w of α-lactalbumin.

7. The method of claim 5, wherein the nutritional formulation further comprises at least one prebiotic.

8. The method of claim 5, wherein the nutritional formulation further comprises vitamins and minerals.

9. A method for providing nutritional support to a preterm infant, wherein the method comprises enterally administering to the preterm infant a nutritional formulation comprising:
an emulsion comprising docosahexaenoic acid, wherein the emulsion is dispersed in an aqueous component comprising an amino acid component comprising i) arginine and alanyl-glutamine dipeptide, or ii) arginyl-glutamine dipeptide;
an emulsifier comprising at least 95% w/w α-lactalbumin, and
arachidonic acid,
wherein the nutritional formulation comprises 0.1 to 1.0 % w/w of the α-lactalbumin, and wherein the formulation is administered in a small volume of up to 2 mL per volume dose.

10. The method of claim 9, wherein the emulsifier comprises at least 98% w/w of α-lactalbumin.

11. The method of claim 9, wherein the nutritional formulation further comprises at least one prebiotic.

12. The method of claim 9, wherein the nutritional formulation further comprises vitamins and minerals.

13. The method of claim 9, wherein the nutritional formulation is administered in a volume dosage of 1 mL, 1.5 mL, or 2 mL.

## Patentansprüche

1. Nährstoffformulierung, umfassend:
eine Emulsion, umfassend Docosahexaensäure, wobei die Emulsion dispergiert ist in einer wässrigen Komponente, umfassend eine Aminosäurekomponente, umfassend i) Arginin und Alanyl-Glutamin-Dipeptid oder ii) Arginyl-Glutamin-Dipeptid;
einen Emulgator, umfassend hochgereinigtes α-Lactalbumin, enthaltend mindestens 95 % w/w α-Lactalbumin, und
Arachidonsäure,
wobei die Nährstoffformulierung 0,1 bis 1,0 % w/w des α-Lactalbumins umfasst und in eine Einzeldosis-Abgabepackung von bis zu 2 ml verpackt ist.

2. Flüssige Nährstoffformulierung nach Anspruch 1, wobei das hochgereinigte α-Lactalbumin mindestens 98 % w/w α-Lactalbumin enthält.

3. Flüssige Nährstoffformulierung nach Anspruch 1, ferner umfassend mindestens ein Präbiotikum.

4. Flüssige Nährstoffformulierung nach Anspruch 1, ferner umfassend Vitamine und Mineralien.

5. Verfahren zur Bereitstellung von Nährstoffunterstützung für ein Subjekt, das Verfahren umfassend die Verabreichung an das Subjekt einer Nährstoffformulierung, umfassend:
eine Emulsion, umfassend Docosahexaensäure, wobei die Emulsion dispergiert ist in einer wässrigen Komponente, umfassend eine Aminosäurekomponente, umfassend i) Arginin und Alanyl-Glutamin-Dipeptid oder ii) Arginyl-Glutamin-Dipeptid; und
einen Emulgator, umfassend hochgereinigtes α-Lactalbumin, enthaltend mindestens 95 % w/w α-Lactalbumin, und
Arachidonsäure,
wobei die Nährstoffformulierung 0,1 bis 1,0 % w/w des α-Lactalbumins umfasst, wobei das Subjekt kleinvolumige enterale Nährstoffunterstützung braucht und wobei das kleine Volumen bis zu 2 ml pro Volumendosis ist.

6. Verfahren nach Anspruch 5, wobei der Emulgator mindestens 95 % w/w α-Lactalbumin, bevorzugt mindestens 98 % w/w α-Lactalbumin, umfasst.

7. Verfahren nach Anspruch 5, wobei die Nährstoffformulierung ferner mindestens ein Präbiotikum umfasst.

8. Verfahren nach Anspruch 5, wobei die Nährstoffformulierung ferner Vitamine und Mineralien umfasst.

9. Verfahren zur Bereitstellung von Nährstoffunterstützung für ein Frühgeborenes, wobei das Verfahren die enterale Verabreichung an das Frühgeborene einer Nährstoffformulierung umfasst, umfassend:
eine Emulsion, umfassend Docosahexaensäure, wobei die Emulsion dispergiert ist in einer wässrigen Komponente, umfassend eine Aminosäurekomponente, umfassend i) Arginin und Alanyl-Glutamin-Dipeptid oder ii) Arginyl-Glutamin-Dipeptid;
einen Emulgator, umfassend mindestens 95 % w/w α-Lactalbumin, und
Arachidonsäure,
wobei die Nährstoffformulierung 0,1 bis 1,0 % w/w des α-Lactalbumins umfasst und wobei die Formulierung in einem kleinen Volumen von bis zu 2 ml pro Volumendosis verabreicht wird.

10. Verfahren nach Anspruch 9, wobei der Emulgator mindestens 98 % w/w α-Lactalbumin umfasst.

11. Verfahren nach Anspruch 9, wobei die Nährstoffformulierung ferner mindestens ein Präbiotikum umfasst.

12. Verfahren nach Anspruch 9, wobei die Nährstoffformulierung ferner Vitamine und Mineralien umfasst.

13. Verfahren nach Anspruch 9, wobei die Nährstoffformulierung in einer Volumendosis von 1 ml, 1,5 ml oder 2 ml verabreicht wird.

## Revendications

1. Formulation nutritionnelle comprenant :
une émulsion comprenant de l'acide docosahexaenoique, dans laquelle l'émulsion est dispersée dans un composant aqueux comprenant un composant d'acide aminé comprenant i) de l'arginine et un dipeptide d'alanyl-glutamine, ou ii) un dipeptide d'arginyl-glutamine ;
un émulsifiant comprenant de l'α-lactalbumine hautement purifiée contenant au moins 95 % en poids/poids d'α-lactalbumine, et
de l'acide arachidonique,
dans laquelle la formulation nutritionnelle comprend de 0,1 à 1,0 % en poids/poids de l'α-lactalbumine et est emballée dans un emballage de fourniture de dose unique allant jusqu'à 2 mL.

2. Formulation nutritionnelle liquide selon la revendication 1, dans laquelle l'α-lactalbumine hautement purifiée contient au moins 98 % en poids/poids d'α-lactalbumine

3. Formulation nutritionnelle liquide selon la revendication 1, comprenant en outre au moins un prébiotique.

4. Formulation nutritionnelle liquide selon la revendication 1, comprenant de plus des vitamines et des minéraux.

5. Méthode pour fournir un support nutritionnel à un sujet, la méthode consistant à administrer au sujet une formulation nutritionnelle comprenant :
une émulsion comprenant de l'acide docosahexaenoique, dans laquelle l'émulsion est dispersée dans un composant aqueux comprenant un composant d'acide aminé comprenant i) de l'arginine et un dipeptide d'alanyl-glutamine, ou ii) un dipeptide d'arginyl-glutamine ; et
un émulsifiant comprenant de l'α-lactalbumine hautement purifiée contenant au moins 95 % en poids/poids d'α-lactalbumine, et
de l'acide arachidonique,
dans laquelle la formulation nutritionnelle comprend de 0,1 à 1,0 % en poids/poids de l'α-lactalbumine, dans laquelle le sujet a besoin d'un petit volume de support de nutrition entérale, et dans laquelle le petit volume est au maximum de 2 mL par dose volumique.

6. Méthode selon la revendication 5, dans laquelle l'émulsifiant comprend au moins 95 % en poids/poids d'α-lactalbumine, de préférence au moins 98 % en poids/poids d'α-lactalbumine.

7. Méthode selon la revendication 5, dans laquelle la formulation nutritionnelle comprend de plus au moins un prébiotique.

8. Méthode selon la revendication 5, dans laquelle la formulation nutritionnelle comprend de plus des vitamines et des minéraux.

9. Méthode pour fournir un support nutritionnel à un enfant prématuré, dans laquelle la méthode comprend l'administration entérale à l'enfant prématuré d'une formation nutritionnelle comprenant :
une émulsion comprenant de l'acide docosahexaenoique, dans laquelle l'émulsion est dispersée dans un composant aqueux comprenant un composant d'acide aminé comprenant i) de l'arginine et un dipeptide d'alanyl-glutamine, ou ii) un dipeptide d'arginyl-glutamine ;
un émulsifiant contenant au moins 95 % en poids/poids d'α-lactalbumine, et
de l'acide arachidonique,
dans laquelle la formulation nutritionnelle comprend de 0,1 à 10 % en poids/poids de l'α-lactalbumine et dans laquelle la formulation est administrée dans un petit volume allant jusqu'à 2 mL par dose volumique.

10. Méthode selon la revendication 9, dans laquelle l'émulsifiant comprend au moins 98 % en poids/poids d'α-lactalbumine.

11. Méthode selon la revendication 9, dans laquelle la formulation nutritionnelle comprend de plus au moins un prébiotique.

12. Méthode selon la revendication 9, dans laquelle la formulation nutritionnelle comprend de plus des vitamines et des minéraux.

13. Méthode selon la revendication 9, dans laquelle la formulation nutritionnelle est administrée dans un dosage volumique de 1 mL, 1,5 mL, ou 2 mL.
